(19) Europäisches Patentamt
European Patent Office
Office européen des brevets

(11) Publication number: **0 185 538 A2**

(12) # EUROPEAN PATENT APPLICATION

(21) Application number: 85309174.2

(22) Date of filing: 17.12.85

(51) Int. Cl.[4]: C 07 D 209/94

(30) Priority: 18.12.84 GB 8431904

(43) Date of publication of application: 25.06.86 Bulletin 86/26

(84) Designated Contracting States: AT BE CH DE FR GB IT LI LU NL SE

(71) Applicant: Kong, Yun Cheung, Dr.
A5 Kingston Building, 8th Floor Kingston Street
Causeway Bay
Hong Kong(HK)

(72) Inventor: Kong, Dr. Yun Cheung
A5 Kingston Building 8th Floor Kingston Street
Causeway Bay(HK)

(72) Inventor: Cheng, Kin Fai
Chemistry Department University of Hong Kong
Pokfulam Road(HK)

(74) Representative: Allam, Peter Clerk et al,
LLOYD WISE, TREGEAR & CO. Norman House 105-109 Strand
London WC2R 0AE(GB)

(54) **The synthesis of indole alkaloids.**

(57) A process for the preparation of yuehchukene, 11β-(3'-indolyl)-7, 9α, 9β-trimethyl-5β, 8, 9, 10β-tetrahydroindano-[2, 3-b-] indole in which 3-(3-methyl-buta-1, 3-dienyl) indole is cyclized in solution in an organic solvent in the presence of silicia gel at temperatures of 60 to 80°C, e.g. by reflux with benzene as the solvent and/or in the presence of an acid catalyst such as trifluoroacetic acid. In turn the 3-(3-methyl-buta-1, 3-dienyl) indole can be prepared from indole-3-carbaldehyde by protecting the nitrogen atom, reaction with the Grignard reagent derived from 3-chloro-2-methylprop-1-ene, followed by deprotection and dehydration.

EP 0 185 538 A2

Croydon Printing Company Ltd.

0185538

# THE SYNTHESIS OF INDOLE ALKALOIDS

This invention relates to the preparation of the indole alkaloid known as yuehchukene.

The indole alkaloid, yuehchukene, with a unique ring structure and potent anti-implantation activity has been isolated from the root of Murraya paniculata (L.) Jack, as disclosed in European Patent Application No. 84304243.3. Examination of the structure of yuehchukene has shown that it is a racemate of 11β-(3'-indolyl)-7,9α, 9β-trimethyl-5β,8,9,10β -tetrahydroindano-[2,3-b-] indole, of the following formula:

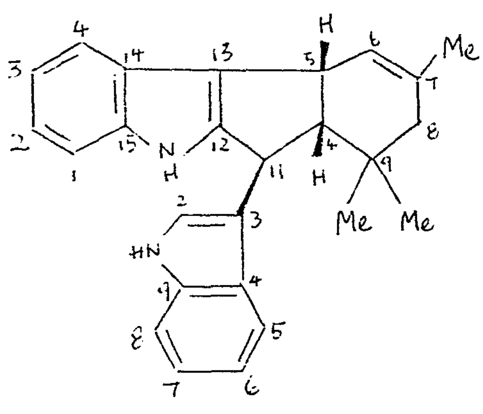

1

The above European Patent Application No. 84304243.3 discloses that yuehchukene can be synthesized by a Diels-Alder condensation of 3-(3-methyl-buta-1,3-dienyl) indole (hereunder MBDI) of the following formula:

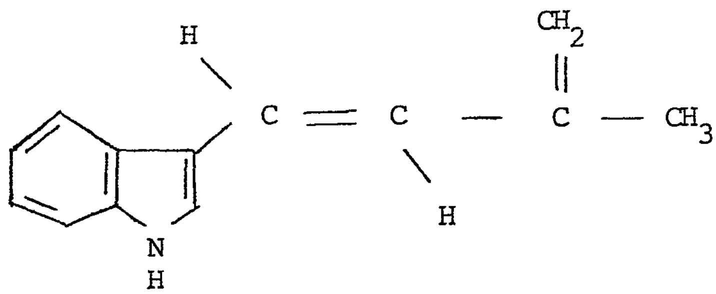

2

An example is presented in which this intermolecular Diels-Alder cyclization is conducted at room temperature in benzene in the presence of silica gel. The resulting slurry is packed into a column which is then rinsed with benzene to yield a fraction containing the desired yuehchukene.

In accordance with the present invention in one aspect, an improved intermolecular Diels-Alder cyclization process is provided whereby very pure and stable yuehchukene may be obtained in better yield. This improved process is characterized by conducting the cyclization of MBDI, in solution in an organic solvent, preferably an aprotic solvent such as benzene in the presence of silica gel at elevated temperatures of 60 to 80°C. Such conditions give an optimum yield with relatively low yields of side reactions.

In one embodiment the reaction is affected under reflux, e.g. in benzene typically for 60 min. At the conclusion of the reaction, the product advantageously is purified by reverse-phase chromatography to yield a stable product in powder form.

In one preferred embodiment the intermolecular cyclization of MBDI is conducted in the presence of an acid catalyst which is easily protonised permitting the reaction to be conducted at a lower temperature and for a shorter time (typically 68°C, 30 min). A particularly suitable acid catalyst is trifluoroacetic acid but acetic acid and hydrochloric acid could also be used; strong mineral acids are not suitable. The product may be purified by normal-phase chromatography or by reverse-phase chromatography as indicated above.

The process of the invention is illustrated by the accompanying flowsheet.

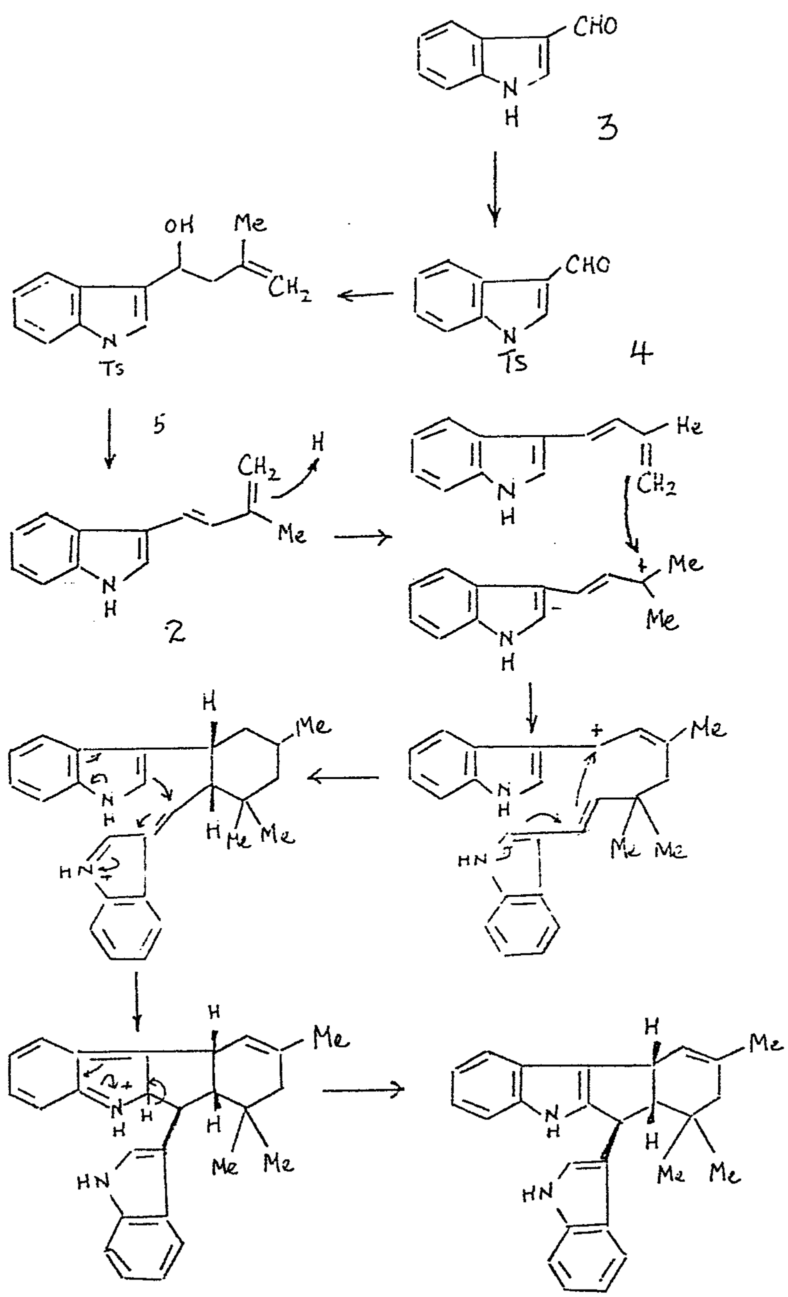

3

4

5

2

The above European Patent Application No. 84304243.3 teaches that the MBDI precursor may be prepared by the aldol condensation of indole-3-carboxyaldehyde with acetone to give 3-(3-oxybutylidenyl)-indole, which upon Wittig olefination with methyltriphenylphosphorium bromide gave the desired MBDI. In accordance with another preferred embodiment, the MBDI is prepared from indole-3-carbaldehyde by protecting the nitrogen atom, e.g. with a p-toluenesulphonyl group, reaction with the Grignard reagent derived from 3-chloro-2-methyprop-1-ene, followed by deprotection and dehydration. This process is relatively simple and each step gives a high yield.

The present invention is further illustrated by the Examples which follow, Example 1 showing the preparation of MBDI and Examples 2 and 3 the synthesis of yuehchukene therefrom.

## Example 1

## Preparation of MBDI

Step I. Preparation of 1-(4-methylbenzenesulphonyl)-indole-3-carbaldehyde, (N-Ts-ICA)

Indole-3-carbaldehyde (29g, 0.1 mole) in dry glyme (200 ml) was added to sodium hydride in 60% mineral oil dispersion (12.3g, 0.15 mole) in dry glyme (30 ml). The mixture was stirred at room temperature under an atmosphere of nitrogen for 1 hour, followed by warming to 60°C for 10 minutes. To this reaction mixture was added

p-toluenesulphonyl chloride (42g, 0.11 mole) and imidazole (1g) in dry glyme (50 ml) at 0°C. The whole was warmed at 60°C for 1 hour. After cooling down with ice bath, the grey precipitate thus formed was filtered off and discarded. The filtrate was poured into a saturated solution of sodium hydrogen carbonate ( 400 ml) at 0°C. The yellow precipitate of the desired product, N-Ts-ICA (4) (50g, 80% yield) was collected and dried in vacuum.

Step II. Preparation of 1-(4-methylbenzenesulphonyl)-3-(1-hydroxy-3-methylbut-3-enyl)-indole, (NTs-3HMBI)

A solution of N-Ts-ICA (19g, 63.5 mmole) and 3-chloro-2-methyl-1-propene (11.3g, 125 mmole) in tetrahydrofuran (300 ml) was added dropwise, to magnesium turning (3g, 125 mmole) in THF (20 ml). After complete addition, the mixture was warmed at 60°C for 1 hour, and reaction mixture was treated with saturated ammonium chloride solution until all solid dissolved. After appropriate working up, a viscous brown oil was obtained. Purification of the crude product by column chromatography on silica gel (500g) eluting with 1:1-petroleum ether (boiling point 60°C) - ethyl ether afforded the desired NTs-3-HMBI (5) as viscous light brown oil (18g, 75% yield). [$\nu_{max}$ 3560, 1650, 1600, 1565, and 895 $cm^{-1}$; $^1H$ n.m.r. ($CDCl_3$), $\delta$ 1.76 (3H, s), 2.28(3H,s), 2.32(1H,br.s), 2.56(2H,d,*J*7Hz), 4.89(1H,br.s), 5.04(H,t,*J*6.56Hz), 4.81(1H,br.s), 7.10-7.39(4H,m), 7.52-7.78(4H,m), and 7.92(1H,m); *m/z* 355, (*M*$^+$)].

Step III. Preparation of 3-(3-methylbuta-1,3-dienyl)indole, MBDI

A solution of NTs-3-HMBI (17g), in 3N aqueous ethanolic (1:8) potassium hydroxide (700 ml) was warmed at 50°C under nitrogen atmosphere for 1 hour. Extracting the reaction product with several portions of diethyl ether (500 ml) and removing the solvent afforded a solid residue. The residue was washed with small amount of absolute ethanol and MBDI thus obtained was a yellow solid (6g, 65% yield). The red solution from the absolute ethanol washing contained MBDI. Silica gel chromatography of this solution afforded further 10% yield of MBDI . [m.p. 130-132°C; $\nu_{max}$ (KBr) 1630, 1605, 965, and 875 $cm^{-1}$; $^1H$ n.m.r. ($CDCl_3$) $\delta$ 2.01(3H,s), 4.98(1H,br.s), 5.05(1H,br.s), 6.75(1H,d,J16.41 Hz), 6.93(1H,d,J16.41 Hz), 7.07-7.40(4H,m), 7.91(1H,m), and 8.05(1H,br.s); m/3 183($M^+$)].

MBDI thus obtained was then subjected to intermolecular Diels-Alder cyclization without further purification according to one of the two methods described below in Examples 2 and 3.

## Example 2

### Synthesis of yuehchukene by reflux cyclization

To 100 g of silica gel (70-230 mesh) in 250 ml benzene, 6.5 g of MBDI was added and stirred until it was completely dissolved and absorbed on the silica gel. The reaction mixture was slowly warmed up to boiling point in 30 min. and kept refluxing for another 60 min. All this

while, the reaction mixture was constantly stirred and flushed with argon. At the end of reaction, benzene was recovered by suction filtration and the silica gel was rinsed exhaustively with diethyl ether until it was white again. The combined filtrate was concentrated to a thick syrup in a rotary evaporator and then loaded on a silica gel column (250 g silica gel, 230-400 mesh, 5 by 20 cm) packed in 30-60° petroleum ether: diethyl ether (1:1). The column was eluted with the same solvent under pressure (nitrogen) with a solvent linear speed of 5 cm/min. (flash column). The first 650 ml eluate was collected and the residue recovered amounted to 30% of the sample loaded on the column. It was then chromatographed in a Waters Prep LC/system 500A unit using a PrepPak 500/$C_{18}$ column (380 g silica gel-$C_{18}$) and eluted with 80% methanol in water at a flow rate of 150 ml/min. A third peak, which was the main peak, leaving the column at a peak retention time of 30 minutes was the yuehchukene peak. For further purification, this peak could be recycled on the same column. Alternatively, it could be collected, concentrated and dried, and then applied to a size B Merck Lobar RP-8 column and eluted with the same solvent (80% methanol in water).

Yuehchukene recovered from reverse-phase chromatography was first concentrated in a rotary evaporator to remove the methanol, then lyophilised to dryness. The final product is a light lemon-yellow powder.

It has the same mass spectrum (70 eV) and $^{1}H$-nuclear magnetic resonance (90 M-Hz) spectrum as the pure natural product. Bioassay with mated female rats, dosing by oral route on pregnancy day 1-2 at 2.5 mg/kg body weight was 100% active in preventing pregnancy. By the same token, the single day/single dose combination is 3 mg/kg dosing on pregnancy day 2.

Yuehchukene prepared by this process was kept at -20°C stored in sealed glass vials and filled with argon. It is stable under these conditions.

The flash column yielded 11% of yuehchukene by weight, as estimated by quantitative HPLC. The reverse-phase columns (500/$C_{18}$ leading to RP-8) yielded 5% of near-pure yuehchukene by weight from MBDI. It was 97% pure.

Example 3

Synthesis of yuehchukene by acid-catalysed cyclization

Yuehchukene can be obtained in a similar process as described above in Example 2 with the following modifications. After the MBDI was completely dissolved and absorbed on the silica gel, 1N trifluoracetic acid (TFA) in benzene was added dropwise to the reaction mixture which was constantly stirred and flushed with argon. The final concentration of TFA in the reaction mixture should reach a molar ratio of MBDI:TFA of 25:1. Increasing the molar ratio to 5:1 can increase the yield of yuehchukene but at the same time the complexity of side products, thus

rendering purification more difficult and the final yield lower. When the addition of TFA was completed, the reaction mixture was slowly warmed to 65°C and maintained at this temperature for 30 min. At the end of reaction, the solvent was recovered by suction filtration and the silica gel was rinsed exhaustively with diethyl ether. The combined filtrate was first partitioned with water, then with 10% $KHCO_3$ in water to remove TFA. After drying over anhydrous magnesium sulphate, it was first processed by the flash column as described in Example 2. The flash column eluate containing yuehchukene was further purified by a Merck Lobar column using 30°-60° petroleum ether:diethyl ether (1:1) as solvent. Yuehchukene was recovered by removing the solvent in a rotary evaporator and then vacuum-pumped to dryness. The final product is a light brown amorphous mass at 10% yield (on a weight basis from MBDI). It has been identified by uv, ir, $^{1}H$-nmr and $^{13}C$-nmr spectra to have the same structure as the pure natural product. It has the same biological potence (100% active at 2.5 mg/kg, dosing p.o. on Preg. day 1-2). Alternatively, the reaction product can be purified by reverse-phase chromatography as in Example 2 which yielded yuehchukene in a powder form.

C L A I M S:

1) A process for the preparation of yuehchukene of the formula:

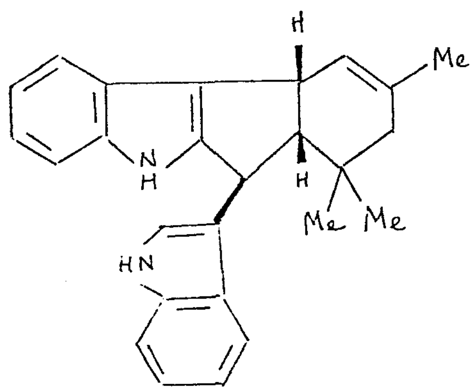

comprising cyclizing 3-(3-methyl-buta-1,3-dienyl) indole in solution in an organic solvent in the presence of silica gel, characterised in that the cyclizing is effected at elevated temperatures of from 60 to 80°C.

2) A process as claimed in Claim 1 characterised in that the cyclizing is effected under reflux.

3) A process as claimed in Claim 1 or Claim 2 characterised in that the cyclizing is effected in the presence of an acid catalyst.

4) A process as claimed in Claim 3 characterised in that the acid catalyst is trifluoroacetic acid.

5) A process as claimed in any preceding claim characterised in that the organic solvent is benzene.

6) A process as claimed in any preceding claim characterised in that the yuehchukene is purified by normal-phase chromatography or by reverse-phase chromatography.

7) A process as claimed in any preceding claim characterised in that the 3-(3-methyl-buta-1,3-dienyl) indole is prepared from indole-3-carbaldehyde by protecting the nitrogen atom, reaction with the Grignard reagent derived from 3-chloro-2-methylprop-1-ene, followed by deprotection and dehydration.